# EUROPEAN PATENT APPLICATION

(11) **EP 0 807 436 A1**
(43) Date of publication of application: **19.11.1997**
(21) Application number: 96900733.5
(22) Date of filing: 22.01.1996
(51) Int. Cl.: A61K 31/725, A61K 35/78

(54) **MUSCULAR DYSTROPHY PROGRESSION DEPRESSANT**

(30) Priority: 23.01.1995 JP 8335/95
(71) Applicant: ZAIDAN HOJIN MINSEI KAGAKU KYOKAI, Koganei-Shi Tokyo 184 (JP)
(72) Inventor: YAMADA, Shigeru, Kanagawa 213 (JP); FUJIMAKI, Masato, Tokyo 184 (JP)
(74) Representative: Dealtry, Brian
(86) International application number: JP9600107
(87) International publication number: WO9622779

(57) **Abstract**

A method for depressing the progression of muscular dystrophy, namely, the destruction of muscular cells and muscular asthenia; and a muscular dystrophy progression depressant which comprises carrageenan.

## Description

### TECHNICAL FIELD

The present invention relates to the inhibition of the progression of muscular dystrophy. In particular, the present invention relates to an inhibitory agent comprising carrageenan for the progression of muscular dystrophy, which inhibits the progression of sarcous denaturation due to muscular dystrophy.

### BACKGROUND OF THE INVENTION

### Background Art

Muscular dystrophy is a genetic disease which causes the progressive denaturation of muscle. This is classified into Duchenne's dystrophy, Becher type dystrophy, facioscapulohumeral muscular dystrophy and myotonic dystrophy depending on the age of the patient affected by dystrophy or the distribution of muscles affected by dystrophy.

Among them, the one which occurs most frequently is said to be Duchenne's muscular dystrophy. The patients affected by the Duchenne's muscular dystrophy who are usually infants generally exhibit the development of physical functions slower than the healthy infants, and the slower development may take place along with growth of the patients until the ages of 5 - 6 years. However, the disease progresses further later, and most of the patients become abatic at an average age of eleven year old and died in myocardiopathy or respiratory insufficiency. Moreover, the progression of the disease often occurs symmetrically.

It is understood that muscular dystrophy is a disease which causes adynamic physical disorder due to the displacement of muscular fibers by adipose tissues or connective tissues. This disease has been investigated in many aspects, and it has been revealed that the patients have a variety of genetic delections in the region Xp21 on the X chromosome and are brought into crisis by the lack of dystrophin which should be coded for at the site. However, no appropriate therapy has been established up to now.

Muscular dystrophy is a genetic disease, and a patient congenitally contracts the disease, which progresses intensively in childhood or at senescense. It is believed that if the progression of the disease is inhibited for a certain period of time, the disease will be successfully inhibited from progression even after the period as well. Thus, a variety of nosotropic therapies, for example with adrenaline, PILOCARLLINE or glycocoll, have been used for inhibiting the progression of the disease. However, these therapies have resulted in only insufficient effects and the methods for inhibiting the progression of muscular dystrophy have not been established, as far as the present inventors know.

By the way, the present inventors have found that myocytes may be proliferated or enlarged by the administration of carrageenan (Japanese Patent Application No. 345428/1993).

### SUMMARY OF THE INVENTION

The present invention relates to the process for inhibiting the progression of muscular dystrophy which has been difficult to be inhibited as described above.

The agent for inhibiting the progression of muscular dystrophy according to the present invention comprises carrageenan.

Also, the process for inhibiting the progression of muscular dystrophy according to the present invention comprises administering carrageenan to an animal affected with muscular dystrophy.

It is possible to inhibit the destruction of myocites and the decline of myodynamia accompanied therewith, and thus it is possible to inhibit the progression of muscular dystrophy.

While the present inventors have found that carrageenan has a function of proliferating or enlarging animal myocites as described above, the present invention is to inhibit the progression of the denaturation of myocites due to muscular dystrophy and thus is broadly understood that it is distinguished from the technique according to the prior invention of which object is to proliferate or enlarge myocites with carrageenan according to the prior invention.

As carrageenan is an edible polysaccharide, it is believed that its oral ingestion is safe for animals and its intramuscular injection is also safe for animals from the experimental results with mice and fowls.

Carrageenan is used for the same objects as agar with its gel formability and viscosity including stabilizing agents of jellies, chocolates and dairy products, emulsifiers of a cod liver oil for pharmaceutical uses, and culture media of bacteria, but it has not been used as an agent for inhibiting the progression of muscular dystrophy as far as the present inventors know. It can be said unexpected that a polysaccharide such as carrageenan has an inhibitory effect for muscular dystrophy.

### DETAILED DESCRIPTION OF THE INVENTION

### [Carrageenan]

Carrageenan occurs naturally as the cell membrane component of red algae such as Chondrus crispus or Chondrus ocellatus in Japan as well as Gigartina ocellatus, and is obtained by subjecting one of these seaweeds to extraction with hot water. It is one of galactanes having sulfate groups.

Carrageenan generally comprises two components of κ-carrageenan which is precipitated with potassium chloride and λ-carrageenan which is not precipitated with potassium chloride.

Among these components, λ-carrageenan is a polymeric compound having a repeating unit represented by the general formula [I] and a molecular weight of about 30,000 - 40,000.

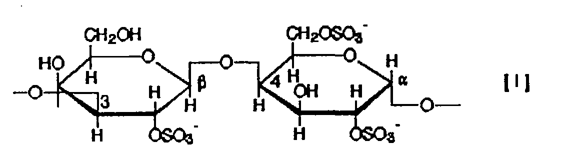

Also, κ-carrageenan is a polymeric compound having a repeating unit represented by the general formula [II] and a molecular weight of about 80,000 - 100,000.

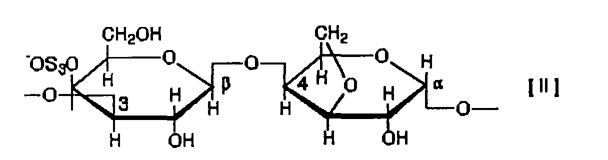

The term "carrageenan" used herein means inclusively the so-called "carrageenan" which comprises the two components of λ-carrageenan and κ-carrageenan derived from the natural products as well as the components *per se*, namely κ-carrageenan and λ-carrageenan, their decomposed products, and mixtures thereof in any ratios. It also includes a variety of salts thereof as far as the effect of the present invention will not be impaired.

The structures of the carrageenan described above are the ones which are generally recognized in the art, but the present invention is not limited to these structures.

Among these carrageenan, the preferred one is λ-carrageenan and/or its decomposed products, particularly λ-carrageenan. If the carrageenan is a mixture of plural spcies of carrageenans, the those having a higher ratio of λ-carrageenan are preferred.

The decomposed product of λ-carrageenan means the hydrolysate of λ-carrageenan having a molecular weight of about 30,000.

### [Carrageenan-containing agent for inhibiting the progression of muscular dystrophy and its administration]

The agent for inhibiting the progression of muscular dystrophy according to the present invention comprises carrageenan and can be prepared in any dosage forms corresponding to its administration routes as far as carrageenan is an effective component.

In other words, the agent for inhibiting the progression of muscular dystrophy according to the present invention may be administered orally or parenterally to humans or animals other than humans. The administration route is preferably parenteral routes such as intramuscular injection, intravenous injection, subcutaneous injection, intrarectal administration, percutaneous administration, and the like, particularly intramuscular injection. The carrageenan-containing agent for inhibiting the progression of muscular dystrophy according to the present invention is thus employed in a variety of dosage forms appropriate for oral or parenteral administrations. For instance, it can be prepared in any of the dosage forms such as oral preparations including tablets, capsules, granules, pills, fine particles and a troche; injections; intrarectal preparations; and water soluble suppositories. These preparations can be prepared by the usual methods with usually empoloyed additives such as an excipient, a filler, a bonding agent, a humectant, a disintegrating agent, a surfactant, a lubricant, a dispersant, a buffer, a preservative, a dissolution aid, an antiseptic, a flavoring agent, an analgesic agent, and a stabilizing agent.

One embodiment of the intramuscular injection comprises 1 mg of carrageenan dissolved in 20 cc of a phosphate buffer.

The dose of carrageenan is appropriately determined in consideration of factors such as routes of administration, ages and sexes of patients, and significant effect is recognized at a dose of 1 µg/kg of the body weight of humans or animals other than humans and maintained for a long period with its single dose.

### BRIEF DESCRIPTION OF THE DRAWINGS

Figure 1 illustrates the variation of muscular weight in the administration of carrageenan to fowls which are not affected by muscular dystrophy (line 412).

Figure 2 illustrates the variation of muscular weight in the administration of carrageenan to fowls which are affected by muscular dystrophy (line 413).

### EXAMPLES

The inhibition of muscular dystrophy according to the present invention is described in more detail with reference to the following examples without limitation thereto.

### Example 1

MDX mice (4 weeks of age; NIPPON KUREA K.K., Japan) which exhibited the symptom of Duchenne's muscular dystrophy was used as experimental animals. A solution of 0.1 g of λ-carrageenan (Wako Pure Chemical Industries, Ltd., Japan) in 20 ml of a phosphate buffer was used as an agent for inhibiting the progression of muscular dystrophy.

A 50 µl portion of the agent for inhibiting the progression of muscular dystrophy was injected into the soleus muscle of the right leg of the MDX mice, and the same amount of the phosphate buffer was injected into the left leg of the same mice through a needle No. 26G.

After 8 weeks of the injections, the animals were slaughtered under anesthetization, and the soleus muscle as the subject muscle was extracted carefully for measuring the weight. The result is shown below.

| | Muscle to which carrageenan was administered | Muscle to which phosphate buffer was administered |
|---|---|---|
| Absolute wet weight (mg) | 12.2 | 10 |
| Wet weight per body weight (mg/10g) | 4.56 | 3.74 |

These muscles were examined on the contraction tension and contraction mode under the following stimulation conditions.

### Stimulation conditions:

- electric stimulating apparatus:: SEN-3201 model by NIPPON KODEN (Japan),
- stimulating electrode:: dipole silver electrode,
- strain gauge:: LTS-500Gf model by KYOWA DENGYOSHA (Japan),
- strain amplifier:: DSA603 model by SHINKOSHA (Japan),
- temperature:: 35 - 36°C.

The measurement was carried out with the muscle fixed at a length so that the maximal contraction is achieved.

### [Measurement of the twitch of muscle]

Muscle contracts in response to each of stimulations, reaches the maximal contraction and then relaxes to the original length. This is called twitch. For the contraction-relaxation cycle, the maximal twitch tension (T), the maximal twitch tension per muscle weight (T/W), the time required for reaching the maximum tension from initiation of contraction (t_{0-MAX}), and the time required for relaxing from the maximal tension to the half level of the maxmal tension (t_{MAX-1/2}) were measured.

| | Muscle to which carrageenan was administered | Muscle to which phosphate buffer was administered |
|---|---|---|
| T (g) | 3.28 | 2.56 |
| T/W (g/mg) | 0.246 | 0.238 |
| t_{0-MAX} (msec) | 18.83 | 19.96 |
| t_{MAX-1/2} (msec) | 26.37 | 21.98 |

The muscle to which carrageenan was administered showed stronger myodynamia than the comparative muscle to which the phosphate buffer was administered, and it also responds more rapidly and maintains contraction for a longer time than the comparative muscle.

### [Strong contraction]

Myodynamia due to strong contraction in response to the stimulations of 40 - 250 Hz to muscle is shown below.

| Stimulaition frequency | Muscle to which carrageenan was administered (g) | Muscle to which phosphate buffer was administered (g) |
|---|---|---|
| 40 Hz | 15.04 | 12.91 |
| 50 Hz | 17.31 | 14.57 |
| 100 Hz | 20.44 | 18.43 |
| 200 Hz | 21.74 | 19.55 |
| 250 Hz | 20.99 | 19.44 |

In strong contraction, the muscle to which carrageenan was administered showed stronger myodynamia than the comparative muscle to which the phosphate buffer was administered.

### Example 2

Muscular dystrophy fowls (line 413, 2 - 4 weeks of age; NIPPON SEIBUTSU KAGAKU KENKYUJO, Japan) were employed as experimental animals. In the muscular dystrophy fowls, pectoralis muscle tends to be affected unlike Duchenne's muscular dystrophy, and the muscles affected tend to have a decreased number of myocytes as well as increased amount of fats thus resulting in whitend and enlarged muscles. Line 413 was used as the fowls which are not affected by muscular dystrophy.

Also, a solution of 0.1 g of λ-carrageenan (Wako Pure Chemical Industries, Ltd.) in 20 ml of a phosphate buffer was used as an agent for inhibiting the progression of muscular dystrophy.

A 1 cc portion of the agent for inhibiting the progression of muscular dystrophy was injected portionwise into several parts of the right pectoralis muscle of the muscular dystrophy fowl line 413, and the same amount of the phosphate buffer into the left pectoralis muscle of the same fowl for comparison. Also, for the line 412 fowl, the agent and the phosphate buffer were injected in the same manner as described above.

After 12 weeks of the injections, the animals were slaughtered under anesthetization, and the pectoralis muscle as the subject muscle was extracted carefully for observation.

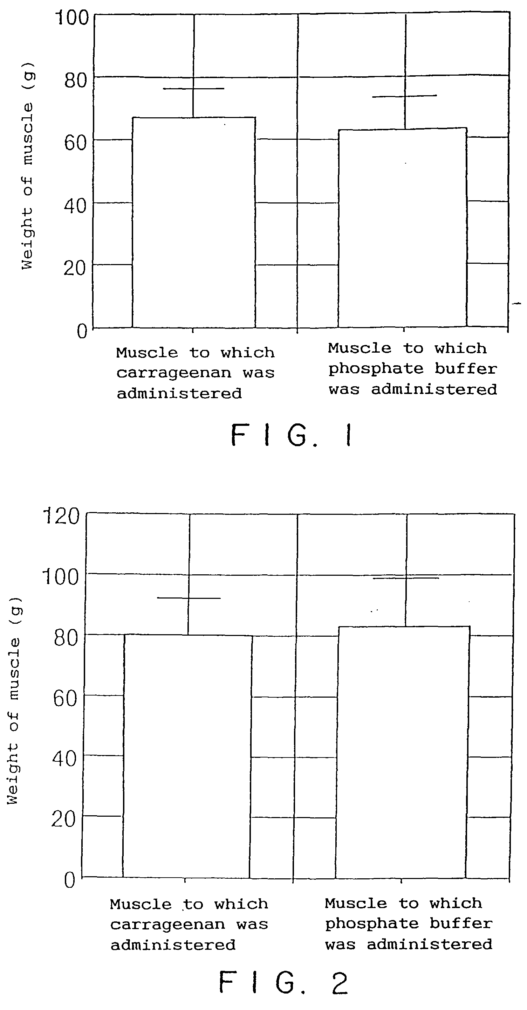

First of all, the weights of the muscle of the line 412 fowls which are not affected by muscular dystrophy are shown in Figure 1. The muscle to which carrageenan was administered was enlarged and grew intensively as compared with the muscle to which the phosphate buffer was administered. In this case, carrageenan showed the effect as the skeletal muscle growing agent.

On the other hand, in muscular dystrophy line 413 fowls, the left pectoralis muscle to which the phosphate buffer was heavier than the right pectoralis muscle to which carrageenan was administered (Figure 2). Furthermore, the left pectoralis muscle as the comparative one had been discolored into white as was usually observed in muscular dystrophy fowls. This is considered due to the denaturation of myocytes into fat or fibrous products by the progression of muscular dystrophy. On the other hand, the right pectoralis muscle to which carrageenan was administered was the reddish one which resembled the muscle of a healthy fowl. This apparently proves that carrageenan not only enlarges myocytes but also works as an agent for inhibiting the progression of muscular dystrophy.

## Claims

1. An agent for inhibiting the progression of muscular dystrophy, which comprises carrageenan.

2. An agent for inhibiting the progression of muscular dystrophy according to claim 1, wherein carrageenan is λ-carrageenan and/or a decomposed product of λ-carrageenan.

3. An agent for inhibiting the progression of muscular dystrophy according to claim 2, wherein carrageenan is λ-carrageenan.

4. An agent for inhibiting the progression of muscular dystrophy according to any one of claims 1 -3, which is in the form of an oral preparation.

5. An agent for inhibiting the progression of muscular dystrophy according to any one of claims 1 -3, which is in the form of a parenteral preparation.

6. An agent for inhibiting the progression of muscular dystrophy according to any one of claims 1 -3, which is in the form of an intramuscular injection.

7. A method of inhibiting the progression of muscular dystrophy, which comprises administering carrageenan to an animal affected by muscular dystrophy.

8. A method of inhibiting the progression of muscular dystrophy according to claim 7, wherein carrageenan is λ-carrageenan and/or a decomposed product of λ-carrageenan.

9. A method of inhibiting the progression of muscular dystrophy according to claim 8, wherein carrageenan is λ-carrageenan.

10. A method of inhibiting the progression of muscular dystrophy according to any one of claims 7 - 9, wherein carrageenan is administered orally.

11. A method of inhibiting the progression of muscular dystrophy according to any one of claims 7 - 9, wherein carrageenan is administered parenterally.

12. A method of inhibiting the progression of muscular dystrophy according to claim 11, wherein carrageenan is administered via intramuscular injection.

13. A use of carrageenan as an agent for inhibiting the progression of muscular dystrophy.

14. A use of carrageenan for the purpose of preparing a pharmaceutical for inhibiting the progression of muscular dystrophy.
